# EUROPEAN PATENT SPECIFICATION

(11) **EP 2 981 616 B1**
(45) Date of publication and mention of the grant of the patent: **31.10.2018**
(21) Application number: 14747830.9
(22) Date of filing: 14.07.2014
(51) Int. Cl.: C12N 15/87

(54) **SOLID PHASE TRANSFECTION OF PROTEINS AND NUCLEIC ACIDS**
VERFAHREN FÜR DIE FESTPHASENTRANSFEKTION VON PROTEINEN UND NUKLEINSÄUREN
PROCÉDÉ DE TRANSFECTION EN PHASE SOLIDE DE PROTÉINES ET D'ACIDES NUCLÉIQUES

(30) Priority: 15.07.2013 EP 13003546
(43) Date of publication of application: 10.02.2016
(73) Proprietor: Universität Heidelberg, 69117 Heidelberg (DE)
(72) Inventor: ERFLE, Holger, 69239 Neckarsteinach (DE); STARKUVIENE-ERFLE, Vytaute, 69239 Neckarsteinach (DE); BULKESCHER, Ruben, 68219 Mannheim (DE)
(74) Representative: Müller-Boré & Partner Patentanwälte PartG mbB
(86) International application number: PCT/EP2014/001924
(87) International publication number: WO 2015/007383

(56) References cited:
- WO-A1-01/20015
- WO-A2-2004/012658
- US-A1- 2003 077 827
- Jürgen Reymann ET AL: "Next-generation 9216-microwell cell arrays for high-content screening microscopy", BioTechniques, 1 October 2009 (2009-10-01), pages 1(877)-5(878), XP055088859, England DOI: 10.2144/000113251 Retrieved from the Internet: URL:http://www.ncbi.nlm.nih.gov/pubmed/198 52772 [retrieved on 2014-09-17]
- ERFLE H ET AL: "Work flow for multiplexing siRNA assays by solid-phase reverse transfection in multiwell plates", JOURNAL OF BIOMOLECULAR SCREENING, vol. 13, no. 7, 1 August 2008 (2008-08-01) , pages 575-580, XP008116949, ISSN: 1087-0571, DOI: 10.1177/1087057108320133 [retrieved on 2008-08-01]
- SELLS M A ET AL: "Delivery of protein into cells using polycationic liposomes", BIOTECHNIQUES, vol. 19, no. 1, 1 July 1995 (1995-07-01), pages 72-78, XP008121593, ISSN: 0736-6205

## Description

The present invention relates to a method for simultaneous solid phase transfection of at least one antibody or fragment thereof and at least one nucleic acid molecule into at least one cell, comprising the step of providing a solid carrier containing at least one spot comprising a transfection reagent, at least one antibody or fragment thereof and the at least one nucleic acid molecule, as well as to a solid carrier containing at least one spot comprising a transfection reagent, at least one antibody or fragment thereof and the at least one nucleic acid molecule, and uses thereof.

Transfection of proteins or nucleic acid molecules into mammalian cells are well-known techniques of introducing proteins or nucleic acid molecules into a cell using a transfection agent. Using these methods proteins can be transferred into cells without the need of introducing and expressing the encoding gene sequence. Further, nucleic acid molecules can be transferred into cells for regulating the expression of genes, e.g. by introducing recombinant genes into a cell using viral vectors or by repressing gene expression by introducing siRNA molecules.

Antibodies are large glycoproteins (150 kDa) that act as a major component of immune response against pathogenic agents (toxins, viruses, bacteria). Their property to specifically recognize and bind an antigen is successfully exploited in the current molecular research to detect protein expression, localization, interaction partners, modifications and many others. Usually, cell lysates or fixed cells are used for these experiments. On the other hand, numerous studies demonstrated that antibodies delivered in living cells correctly recognize their intracellular antigens and, by this, induce specific phenotype changes. Antibodies are also widely used for molecular diagnostics. One of the best developed applications is the detection of antibodies that were produced upon infection (e.g., Lyme disease) or in case of the autoimmune diseases (e.g., Lupus erythematosus) in the blood serum.

Recently, monoclonal antibodies were proven to be useful for therapy of oncological and immunological diseases, and their market is growing exponentially in the last decade. For instance, by targeting specific cell surface receptors, expressed in altered, but not in healthy cells, antibodies can prevent proliferation of cancer cells (e.g. Cetuximab for treating EGFR expressing metastatic colorectal cancer, Herceptin for Her2 over-expressing breast cancer). Applicability of antibody-based therapy, however, has still many hurdles to take; such as the expression level of the antigen does not always predict a response to the antibody-based drug indicating the complexity of the mechanisms involved.

Functional studies and diagnostic application of antibodies are still hindered by their non-ability to cross the plasma membrane (PM) of cells. Early experiments with antibody cellular delivery were largely based on microinjection - a precise tool, but needing an intensive training and barely not scalable to large-scale applications. Other methods include electroporation, usage of membrane permeable peptides, coated beads and other. Lately, more efficient transfection of proteins by lipid-, peptide- and polymer-based reagents has become feasible and is utilized for direct transfection of peptides, proteins and antibodies.

In this context, WO 2004/012658 A2 describes a method for performing surface-mediated protein delivery into living cells as well as a method for fabricating protein-transfected cell cluster arrays. Further, US 2003/0077827 A1 describes a method of transfecting cells using surface-immobilized transfection complexes comprising a nucleic acid.

Solid-phase transfection of proteins and nucleic acids which offers an enormous advance for cell biological assay development, combinatoric high-throughput transfections and by enhanced transfection efficiency multiplexing of different cell lines and assays have not been described yet in literature.

Thus, the problem underlying the present invention is to provide new means for an efficient transfection of proteins and nucleic acid molecules into cells.

The solution to the above technical problem is achieved by the embodiments characterized in the claims.

In particular, the present invention relates to the following items:
1. A method for simultaneous solid phase transfection of at least one protein and at least one nucleic acid molecule into at least one cell, wherein the method comprises the steps of
   (a) providing a solid carrier containing at least one spot comprising a transfection reagent, at least one protein and at least one nucleic acid molecule;
   (b) bringing the spot in step (a) into contact with at least one cell,
   wherein the protein is an antibody or a fragment thereof, and
   wherein said solid carrier is produced by
   (i-α) coating/spotting at least part of the solid carrier with the at least one nucleic acid molecule and a fraction of the transfection mixture first and then
   (ii- α) coating/spotting on the part of the solid carrier being coated/spotted with the at least one nucleic acid molecule and this fraction of the transfection mixture the at least one protein and the remaining fraction of the transfection mixture,
   or
   (i-β) coating/spotting at least part of the solid carrier with the at least one protein and a fraction of the transfection mixture first and then
   (ii- β) coating/spotting on the part of the solid carrier being coated/spotted with the at least one protein and this fraction of the transfection mixture the at least one nucleic acid molecule and the remaining fraction of the transfection mixture.
2. The method of item 1, wherein the transfection reagent and the at least one protein and/or the at least one nucleic acid molecule in the at least one spot are dried or frozen.
3. The method of item 1 or 2, wherein the cell is a cell derived from a cell culture or a cell derived from a sample taken from an individual.
4. The method according to any of the above items, further comprising step (c) incubating the at least one cell on the spot.
5. The method according to item 4, wherein the at least one cell is incubated for 3 to 72 hours.
6. The method according to any of the above items, wherein the at least one nucleic acid molecule is selected from the group consisting of siRNA, miRNA, gRNA, cDNA, LNA, and a viral transfection vector, wherein the siRNA molecule optionally contains an antisense strand.
7. The method according to any of the above items, wherein the solid carrier has at least two spots and wherein at least two spots comprise at least two different proteins and/or at least two different nucleic acid molecules.
8. The method according to any of the above items, wherein the solid carrier has at least two different spots and wherein at least two spots are brought into contact with at least two different cell types.
9. A solid carrier containing at least one spot comprising a transfection reagent, at least one protein and at least one nucleic acid molecule,
   wherein the protein is an antibody or a fragment thereof, and
   wherein said solid carrier is produced by
   (i- α) coating/spotting at least part of the solid carrier with the at least one nucleic acid molecule and a fraction of the transfection mixture first and then
   (ii- α) coating/spotting on the part of the solid carrier being coated/spotted with the at least one nucleic acid molecule and this fraction of the transfection mixture the at least one protein and the remaining fraction of the transfection mixture,
   or
   (i- β) coating/spotting at least part of the solid carrier with the at least one protein and a fraction of the transfection mixture first and then
   (ii- β) coating/spotting on the part of the solid carrier being coated/spotted with the at least one protein and this fraction of the transfection mixture the at least one nucleic acid molecule and the remaining fraction of the transfection mixture.
10. The solid carrier of item 9, containing 9 to 225 spots per cm².
11. The solid carrier of item 9 or 10, wherein each spot has a diameter of 5 to 750 µm.
12. The solid carrier according to any of the above items, wherein the at least one nucleic acid molecule is selected from the group consisting of siRNA, miRNA, gRNA, cDNA, LNA, and a viral transfection vector, wherein the siRNA molecule optionally contains an antisense strand.
13. The solid carrier according to any of items 9 to 12, wherein the transfection reagent and the at least one protein and/or the at least one nucleic acid molecule in the at least one spot are dried or frozen.
14. Use of the solid carrier according to any of items 9 to 13 in the transfection of at
   least one protein into at least one cell.

Further, described herein is a method for solid phase transfection of at least one protein and at least one nucleic acid molecule into at least one cell, wherein the method comprises the steps of
(a) providing a solid carrier containing at least one spot comprising a transfection reagent, at least one protein and at least one nucleic acid molecule;
(b) bringing the spot in step (a) into contact with at least one cell.

Accordingly, in step (a) of the method described herein, a transfection mixture comprising a transfection reagent, at least one protein and at least one nucleic acid molecule on the solid carrier is provided in at least one spot of a solid carrier. In a preferred embodiment, the transfection mixture further comprises gelatine and/or matrigel.

In the method described herein, method steps (a) and (b) can be carried out in any order. In a preferred embodiment, first step (a) is carried out and then step (b) is carried out. In another preferred embodiment, first step (b) is carried out and then step (a) is carried out. In case step (b) is carried out first and then step (a) is carried out, then the transfection mixture is dried in order to fulfil the requirement of a solid phase transfection.

In a preferred embodiment, the method described herein is a method for the simultaneous solid phase transfection of at least one protein and at least one nucleic acid molecule into at least one cell.

As described herein, several ways of solid phase transfection can be addressed.

In a preferred embodiment described herein, at least part of the solid carrier is coated with the transfection mixture comprising a transfection reagent, at least one protein and at least one nucleic acid molecule prior to step (a). This can be accomplished for example by diluting the transfection mixture with water to the desired concentration and then pipetting the transfection mixture on the solid carrier, e.g. into wells of a multiwell-plate (for example 384-well plate, 96-well plate, one well). Then the diluted mixture can be evenly distributed in the wells, optionally followed by a drying step. In a preferred embodiment the drying is carried out using a centrifugal evaporator or by freeze drying. After the drying, the solid carrier contains, on the bottom, a thin layer of the desired transfection complexes. This embodiment has the advantage that more cells per transfection approach can be analyzed based on the fact that one transfection approach in 384-well covers 10 mm².

In another preferred embodiment described herein, the transfection mixture comprising a transfection reagent, at least one protein and at least one nucleic acid molecule is spotted on at least part of the solid carrier, for example with a small metal pin, prior to step (a). This procedure can be followed by a drying step to provide longer storage by a longer stability of the transfection complexes, for example by freeze-drying. After the cell seeding only the cells which grow on the spotted regions take up the complexes, cells in other regions do not take up the complexes. This embodiment has the advantage that more and/or different transfection approaches can be prepared on one solid carrier, because less space is needed. For example one transfection approach on a 400 µm-spot covers 1.26 x10⁻⁷ m².

In another preferred embodiment described herein, at least part of the solid carrier is coated with a fraction of the transfection mixture first and then at least part of the solid carrier being coated with this fraction of the transfection mixture is spotted with the remaining fraction of the transfection mixture prior to step (a). In one embodiment at least part of the solid carrier is coated with the at least one nucleic acid molecule and/or the transfection reagent first, optionally dried, and then the at least one protein and/or the transfection reagent is spotted on the part of the solid carrier being coated with the at least one nucleic acid molecule and/or the transfection reagent. The at least one protein may be the same for every spot or different. This may provide a solid carrier being coated with at least one nucleic acid molecule, which may be stored, wherein the transfection mixture is completed before carrying out transfection with either the same protein or with different proteins for at least part of the spots resulting in a different transfection mixture in different spots on the solid carrier. In another embodiment at least part of the solid carrier is coated with the at least one protein and/or the transfection reagent first, optionally dried, and then the at least one nucleic acid molecule and/or the transfection reagent is spotted on at least part of the solid carrier being coated with the at least one protein and/or the transfection reagent. The at least one nucleic acid molecule may be the same for every spot or different.

In another preferred embodiment described herein, at least part of the solid carrier is spotted with a fraction of the transfection mixture first and then at least part of the solid carrier being spotted with this fraction of the transfection mixture is coated with the remaining fraction of the transfection mixture prior to step (a). In one embodiment at least part of the solid carrier is spotted with the at least one nucleic acid molecule and/or the transfection reagent first, optionally dried, and then the at least one protein and/or the transfection reagent are coated on at least part of the solid carrier being spotted with the at least one nucleic acid molecule and/or the transfection reagent. The at least one nucleic acid molecule may be the same for every spot or different. This may provide a solid carrier being spotted with at least one nucleic acid molecule, which may be stored, wherein the transfection mixture is completed before carrying out transfection with the at least one protein in at least part of the spots of the solid carrier. In another embodiment at least a part of the solid carrier is spotted with the at least one protein and/or the transfection reagent first, optionally dried, and then the at least one nucleic acid molecule and/or the transfection reagent are coated on at least part of the solid carrier being spotted with the at least one protein and/or the transfection reagent. The at least one protein may be the same for every spot or different.

In another preferred embodiment described herein, at least part of the solid carrier is spotted with a fraction of the transfection mixture first and then at least part of the solid carrier being spotted with this fraction of the transfection mixture is spotted with the remaining fraction of the transfection mixture prior to step (a). In one embodiment at least a part of the solid carrier is spotted with the at least one nucleic acid molecule and/or the transfection reagent first, optionally dried, and then the at least one protein and/or the transfection reagent is spotted on at least part of the solid carrier being coated with the at least one nucleic acid molecule and/or the transfection reagent. The at least one protein and the at least one nucleic acid molecule may be the same for every spot or different. This may provide a solid carrier being spotted with at least one nucleic acid molecule, which may be stored, wherein the transfection mixture is completed before carrying out transfection with either the same protein or with different proteins resulting in a different transfection mixture in different spots on the solid carrier. In another embodiment at least part of the solid carrier is spotted with the at least one protein and/or the transfection reagent first, optionally dried, and then the at least one nucleic acid molecule and/or the transfection reagent is spotted on at least part of the solid carrier being coated with the at least one protein and/or the transfection reagent. The at least one protein and the at least one nucleic acid molecule may be the same for every spot or different.

In a preferred embodiment described herein, the transfection mixture is mixed together in the at least one spot of the solid carrier. In another preferred embodiment, the transfection mixture is mixed together before being applied to the at least one spot of the solid carrier. In a particularly preferred embodiment, the transfection mixture is mixed together before being applied to the at least one spot of the solid carrier and complex of the transfection agent with the at least one protein and/or the at least one nucleic acid molecule is formed before the complex is applied to the at least one spot of the solid carrier.

In a preferred embodiment, the at least one protein and/or the at least one nucleic acid molecule is pre-complexed with the transfection reagent in the spot in step (a) of the method described herein. In another preferred embodiment, the at least one protein and/or the at least one nucleic acid molecule is pre-complexed with the transfection reagent before being brought into contact with the spot in step (a) of the method described herein. In another preferred embodiment, a fraction of the transfection mixture is pre-complexed before being brought into contact with the spot in step (a) of the method described herein, while the remaining fraction of transfection mixture is brought into contact with the pre-complexed fraction before step (a).

According to the present disclosure, the term "spot" refers to an area on the surface of the solid carrier. A spot on the solid carrier may have unrestricted size and even include the complete surface of the solid carrier. The reagents may be brought at the spot on the surface of the solid carrier by various methods, including spotting with a needle, for example having a diameter of 150 to 600 µm, and including coating, wherein the spot covers at least part of the surface area of the solid carrier, preferably all of the surface of the solid carrier.

According to the present disclosure, the term "protein" does not underlie a specific restriction and may include any protein, protein complex or polypeptide, including recombinant proteins, protein complexes and polypeptides either isolated from a natural source or obtained via recombinant DNA technology, or a biologically active derivative thereof.

As used herein, the term "biologically active derivative of a protein" includes any derivative of a protein, protein complex or polypeptide having substantially the same functional and/or biological properties of the protein such as binding properties, and/or the same structural basis, such as a peptidic backbone. The polypeptide sequences of the functionally active derivatives may contain deletions, additions and/or substitution of amino acids whose absence, presence and/or substitution, respectively, do not have any substantial negative impact on the activity of the polypeptide, e.g. amino acids which are located in a part of the polypeptide sequence that does not contribute to the biological activity of the protein. Minor deletions, additions and/or substitutions of amino acids of the respective polypeptide sequences which are not altering the biological activity of said polypeptide are also included in the present application as biologically active derivatives.

A protein obtained from a natural source may be any protein isolated from a natural source, like for example a body fluid derived from a mammal. In a preferred embodiment of the present application, the mammal is selected from the group consisting of mouse, human, rat, cat, dog, and monkey.

The protein described herein may be produced by any method known in the art. This may include any method known in the art for the production of recombinant DNA by genetic engineering, e.g. via reverse transcription of RNA and/or amplification of DNA. This includes methods which comprise the recombinant production of the protein.

The recombinant protein used herein may be produced by any method known in the art. This may include any method known in the art for (i) the production of recombinant DNA by genetic engineering, e.g. via reverse transcription of RNA and/or amplification of DNA, (ii) the introduction of recombinant DNA into prokaryotic or eukaryotic cells by transfection, e.g. via electroporation or microinjection, (iii) the cultivation of said transformed cells, e.g. in a continuous or batchwise manner, (iv) the expression of the protein, e.g. constitutive or upon induction, and (v) the isolation of the protein, e.g. from the culture medium or by harvesting the transformed cells, in order to (vi) obtain purified recombinant protein, e.g. via anion exchange chromatography or affinity chromatography.

For example, the recombinant DNA coding for the protein, e.g. a plasmid, may also contain a DNA sequence encoding a selectable marker for selecting the cells which have been successfully transfected with the plasmid. In an example, the plasmid may also confer resistance to a selectable marker, e.g. to the antibiotic drug G418, by delivering a resistance gene, e.g. the neo resistance gene conferring resistance to G418.

The production of the protein may include any method known in the art for the introduction of recombinant DNA into cells, which might be mammalian cells, bacterial cells or insect cells, by transfection, e.g. via electroporation or microinjection. For example, the recombinant expression of the protein can be achieved by introducing an expression plasmid containing the human the protein encoding DNA sequence under the control of one or more regulating sequences such as a strong promoter, into a suitable host cell line by an appropriate transfection method resulting in cells having the introduced sequences stably integrated into the genome. The calcium-phosphate co-precipitation method is an example of a transfection method which may be used herein.

The production of the protein may also include any method known in the art for the cultivation of said transformed cells, e.g. in a continuous or batchwise manner, and the expression of the protein e.g. constitutive or upon induction. In one specific example, the nucleic acid coding for the protein contained in the host organism described herein is expressed via an expression mode selected from the group consisting of induced, transient, and permanent expression. Any expression system known in the art or commercially available can be employed for the expression of a recombinant nucleic acid encoding the protein, including the use of regulatory systems such as suitable, e.g. controllable, promoters, enhancers etc.

The production of the protein may also include any method known in the art for the isolation of the protein, e.g. from the culture medium or by harvesting the transformed cells. For example, the protein-producing cells can be identified by isolating single-cell derived populations i.e. cell clones, via dilution after transfection and optionally via addition of a selective drug to the medium. After isolation the identified cell clones may be cultivated until confluency in order to enable the measurement of the protein content of the cell culture supernatant by enzyme-linked immuno-sorbent assay (ELISA) technique.

Additionally, the production of the protein may include any method known in the art for the purification of the protein, e.g. via anion exchange chromatography or affinity chromatography. In one preferred embodiment the protein can be purified from cell culture supernatants by semi-affinity calcium-dependent anion exchange chromatography, e.g. in an endotoxin-free system. The purified protein may be analyzed by methods known in the art for analyzing recombinant proteins, e.g. the ELISA technique. In addition, the protein integrity and activity may be assessed. It is within the knowledge of a person skilled in the art to choose the optimal parameters, such as buffer system, temperature and pH for the respective detection system to be used.

In one specific example, the protein transfected into a cell according to the present disclosure is expressed in a host cell type with the ability to perform posttranslational modifications. The ability to perform posttranslational modifications of the protein expressing host cell lines may be for example analyzed by mass-spectrometric analysis.

The host cell type used for the recombinant production of the protein may be any bacterial or mammalian cell, preferably with the ability to perform posttranslational modifications of the protein. There is no particular limitation to the media, reagents and conditions used for culturing the cells in the cell culture used for the recombinant protein production of including culturing the cells in a continuous or batchwise manner. The desired protein which has been expressed by the cells of the and which, dependent on the transfection/vector-system used, is contained in the cells or secreted into the medium for culturing cells, can be isolated/recovered from cell culture using methods known in the art.

As used herein, the term "nucleic acid molecule" does not underlie a specific restriction and may include any nucleic acid molecule of any length, including polynucleotides, oligonucleotides, aptamers, deoxyribonucleic acid (DNA), ribonucleic acid (RNA), and artificial nucleic acid analogs, like morpholinos, bridged nucleic acid (BNA), peptide nucleic acid (PNA), locked nucleic acid (LNA), glycol nuclei acid (GNA), and therose nucleic acid (TNA). In a preferred embodiment described herein, the at least one nucleic acid molecule is selected from the group consisting of DNA, mRNA, siRNA, miRNA, sgRNA, gRNA, cDNA, DNA aptameres, viral transfection vectors, and morpholino oligomers.

The siRNA molecule can be any short double stranded RNA, preferably of 15 to 30 nucleotides, more preferably of 19 to 25, most preferably of 21 to 23 nucleotides length. The siRNA may contain modifications within the double-stranded region, including modifications of nucleobases and/or modifications of the backbone, as well as overhangs at the 3' and/or 5' end of the sense and/or antisense strand.

The cDNA molecule can be any cDNA molecule known in the art. Methods for obtaining cDNA by synthezing DNA using a messenger RNA (mRNA) as a template employing the reverse transciptase function of a DNA polymerase are known to a person skilled in the art. In a preferred embodiment, the cDNA molecule is a double-stranded DNA molecule having a length form 20 bp to 15000 bp, more preferably from 40 bp to 6000 bp, more preferably from 100 bp to 4000 bp.

The term nucleic acid molecule as used herein includes recombinant and synthetic nucleic acid molecules as well as nucleic acid molecules which are isolated from a natural source, including biologically active derivatives thereof.

As used herein, the term "biologically active derivative of a nucleic acid molecule" includes any derivative of a nucleic acid molecule having substantially the same functional and/or biological properties of the at least one nucleic acid molecule such as coding properties, and/or the same structural basis, such as a nucleotide backbone. The nucleotide sequences of the functionally active derivatives may contain deletions, additions and/or substitution of nucleotides whose absence, presence and/or substitution, respectively, do not have any substantial negative impact on the activity of the nucleic acid molecule, e.g. nucleotides which are located in a part of the nucleotide sequence that does not contribute to the biological activity of the nucleic acid molecule. Minor deletions, additions and/or substitutions of nucleotides of the respective nucleotide sequences which are not altering the biological activity of said nucleic acid molecule are also included in the present application as biologically active derivatives.

A nucleic acid molecule obtained from a natural source may be any nucleic acid molecule isolated from a natural source, like for example a tissue or cell sample derived from a mammal. In a preferred embodiment of the present application, the mammal is selected from the group consisting of mouse, human, rat, cat, dog, and monkey.

The nucleic acid molecule described herein may be produced by any method known in the art. This may include any method known in the art for the production of recombinant DNA by genetic engineering, e.g. via reverse transcription of RNA and/or amplification of DNA. This includes methods which comprise the recombinant production of the nucleic acid molecule.

The recombinant nucleic acid molecule used according to the present disclosure may be produced by any method known in the art. This may include any method known in the art for (i) the production of recombinant DNA by genetic engineering, e.g. via reverse transcription of RNA and/or amplification of DNA, (ii) the introduction of recombinant DNA into prokaryotic or eukaryotic cells by transfection, e.g. via electroporation or microinjection, (iii) the cultivation of said transformed cells, e.g. in a continuous or batchwise manner, and (iv) the isolation of the nucleic acid molecule, e.g. by harvesting the transformed cells, in order to (v) obtain purified recombinant nucleic acid molecule, e.g. via anion exchange chromatography or affinity chromatography.

For example, the recombinant nucleic acid molecule, e.g. a plasmid, may also contain a DNA sequence encoding a selectable marker for selecting the cells which have been successfully transfected with the plasmid. In an example, the plasmid may also confer resistance to a selectable marker, e.g. to the antibiotic drug G418, by delivering a resistance gene, e.g. the neo resistance gene conferring resistance to G418.

The production of the nucleic acid molecule may include any method known in the art for the introduction of recombinant DNA into cells, which might be mammalian cells, bacterial cells or insect cells, by transfection, e.g. via electroporation or microinjection. The production of the nucleic acid molecule may also include any method known in the art for the cultivation of said transformed cells, e.g. in a continuous or batchwise manner

The production of the nucleic acid molecule may also include any method known in the art for the isolation of cells containing the nucleic acid molecule, e.g. from the culture medium or by harvesting the transformed cells. For example, the nucleic acid molecule containing cells can be identified by isolating single-cell derived populations i.e. cell clones, via dilution after transfection and optionally via addition of a selective drug to the medium. After isolation the identified cell clones may be cultivated until confluency.

Protocols for recombinant DNA isolation, cloning and sequencing are well known in the art. Further, methods for the production and isolation of RNA molecules are well known in the art.

As used herein, the term "cells" means a generic term and encompass the cultivation of individual cells, tissues, organs, insect cells, avian cells, mammalian cells, bacterial cells, primary cells, continuous cell lines, stem cells and/or genetically engineered cells, such as recombinant cells expressing a hetereologous polypeptide or protein and/or containing a recombinant nucleic acid molecule and/or stable expressing cells.

The term "solid carrier" does not have any specific limitations, and relates, for example, to glas or an insoluble polymer material, which can be an organic polymer, such as polyamide or a vinyl polymer (e.g. poly(meth)acrylate, polystyrene and polyvinyl alcohol, or derivatives thereof), a natural polymer such as cellulose, dextrane, agarose, chitin and polyamino acids, or an inorganic polymer, such as glass or metallohydroxide. The solid carrier can be in the form of a microcarrier, particles, membranes, strips, paper, film, or plates, such as microtiter plates or microarrays. The term "microarray" as used herein may mean any arrangement of the spots in addressable locations on a support resulting in a so-called "biochip". In a preferred embodiment the solid carrier is a coated solid carrier, for example with collagen, fibronectine, GAPS (Gamma Amino Propyl Silane), sucrose, Silane, Matrigel or polylysine or pretreated with diluted NaOH.

In preferred embodiment, the solid carrier is coated with a cell-adhesive agent, coated with a cell-repelling agent, or uncoated.

In preferred embodiment described herein, the solid carrier is a cell culture device. In another preferred embodiment, the solid carrier is a plate having wells or chambers. In a particularly preferred embodiment, the solid carrier is a 1 well plate, a 2 well plate, a 4 well plate, an 8 well plate, a 12 well plate, a 16 well plate, a 24 well plate, a 32 well plate, a 48 well plate, a 64 well plate, a 96 well plate a 384 well plate or 1536 well plate.

In a preferred embodiment of the method described herein, at least one spot comprising a transfection reagent, at least one protein and at least one nucleic acid molecule on the solid carrier in step (a) further contains a sugar, preferably a nonreducing disaccharide, preferably trehalose or sucrose. Thus, in a preferred embodiment, the transfection mixture in the spot comprising a transfection reagent, at least one protein and at least one nucleic acid molecule on the solid carrier further contains a sugar, preferably sucrose. The sugar may be present in a fluid in any suitable concentration, preferably 0.2 to 0.8 M, more preferably 0.3 to 0.6 M, most preferably 0.4 M.

Further, in another preferred embodiment described herein, the transfection mixture in at least one spot comprising a transfection reagent, at least one protein and at least one nucleic acid molecule on the solid carrier further contains a cell culture medium, preferably, a low serum cell culture medium, most preferably OptiMEM produced by Invitrogen. In another preferred embodiment, the cell culture medium is a serum-free medium, preferably a protein-free medium.

In a more preferred embodiment described herein, the transfection mixture in at least one spot comprising a transfection reagent, at least one protein and at least one nucleic acid molecule on the solid carrier further contains a sugar as defined above being dissolved in a cell culture medium as defined above. In a most preferred embodiment, the spot contains a solution of 0.4 M sucrose in a low serum cell culture medium, most preferably OptiMEM produced by Invitrogen.

In one embodiment described herein, the transfection mixture in at least one spot comprising a transfection reagent, at least one protein and at least one nucleic acid molecule on the solid carrier and optionally the sucrose and/or cell culture medium are incubated after they have been applied to the spot on the solid carrier in step (a). Therefore, in a preferred embodiment, the method described herein contains after step (a) and before step (b) an additional step (a') of incubating the transfection reagent and the at least one protein and the at least one nucleic acid molecule and optionally the sucrose and/or cell culture medium for at least 1 minute, preferably, 2 to 360 minutes, more preferably 5 to 60 minutes, more preferably 10 to 40 minutes, most preferably 15 to 30 minutes. In a preferred embodiment, the incubation is carried out between 4°C to 40°C, preferably between 15°C and 37°C, more preferably between 17°C and 27°C, and most preferably at room temperature.

Therefore, also described herein is a method for solid phase transfection of at least one protein and at least one nucleic acid molecule into at least one cell, wherein the method comprises the steps of
(a) providing a solid carrier containing at least one spot comprising a transfection reagent and at least one protein and at least one nucleic acid molecule;
(a') incubating the transfection reagent and the at least one protein and the at least one nucleic acid molecule; and
(b) bringing the spot in step (a) into contact with at least one cell.

Step (a') may further contain the incubation of the transfection reagent and the at least one protein and the at least one nucleic acid molecule with sucrose and/or cell culture medium as defined above.

In a preferred embodiment described herein, the transfection mixture in at least one spot comprising a transfection reagent, at least one protein and at least one nucleic acid molecule on the solid carrier further contains a glycoprotein, preferably a high-molecular weight glycoprotein, most preferably fibronectin. In a preferred embodiment, the glycoprotein is mixed with gelatin. In a preferred embodiment, the mixture contains 0.1 % to 5%, more preferably 0.2 to 3%, more preferably 0.5 to 1.5% and most preferably 1% glycoprotein. In a preferred embodiment, the mixture contains 0.005% to 2%, more preferably, 0.05 % to 1 %, more preferably 0.1 to 0.5 %, and most preferably 0.2 % gelatin.

In a preferred embodiment of the method described herein, the method contains after step (a) and before step (b) a step wherein the mixture of the glycoprotein and the gelatin as defined above is added to the transfection mixture in a further step (a").

Therefore, also described herein is a method for solid phase transfection of at least one protein and at least one nucleic acid molecule into at least one cell, wherein the method comprises the steps of
(a) providing a solid carrier containing at least one spot comprising a transfection reagent and at least one protein and at least one nucleic acid molecule;
(a") adding a glycoprotein or a mixture of a glycoprotein and gelatin to the spot; and
(b) bringing the spot in step (a) into contact with at least one cell.

In a most preferred embodiment described herein, the method comprises two additional steps, namely step (a') as defined above and step (a") as defined above. In this embodiment step (a") is carried out after step (a').

Therefore, also described herein is a method for solid phase transfection of at least one protein and at least one nucleic acid molecule into at least one cell, wherein the method comprises the steps of
(a) providing a solid carrier containing at least one spot comprising a transfection reagent and at least one protein and at least one nucleic acid molecule;
(a') incubating the transfection reagent and the at least one protein and the at least one nucleic acid molecule;
(a") adding a glycoprotein or a mixture of a glycoprotein and gelatin to the spot; and
(b) bringing the spot in step (a) into contact with at least one cell.

The transfection reagent may be any commercially available transfection agent which is suitable for transfecting proteins into cells. The term "transfection agent" is not limited to agents which are designated for transfecting proteins and/or nucleic acid molecules into cells, but also comprises agents which are designated to transfect other molecules into cells, like for example DNA, RNA, siRNA, proteins or antibodies. Any transfection agent, which does not prevent the transfection of proteins and nucleic acid molecules into cells, is a transfection agent according to the term "transfection agent" as used herein. In a preferred embodiment, the transfection agent is selected from the group consisting of Novagen ProteoJuice, OZ Biosciences DreamFect Gold, ibidi Torpedo DNA, Peqlab PeqFect siRNA, Invitrogen Lipofectamine 2000, Invitrogen Lipofectamine RNAiMAX and Gelantis GeneSilencer.

In step (b) of the method described herein, at least one cell is brought into contact with the transfection mixture in the spot comprising a transfection reagent, at least one protein and at least one nucleic acid molecule on the solid carrier. In this step the transfection of the at least one protein and the at least one nucleic acid molecule into the at least one cell is carried out. It is within the knowledge of the person skilled in the art to choose the optimal parameters, such as buffer system, temperature and pH for the respective fluid to be used for step (b).

According to the present disclosure, the transfection mixture in the spot comprising a transfection reagent, at least one protein and at least one nucleic acid molecule on the solid carrier may further contain any salts or buffer agents which do not prevent the transfection of the protein and the nucleic acid molecule into the cell.

In a preferred embodiment described herein, the at least one cell is incubated on the spot after step (b). The incubation on the spot might be of use for the transfection of the protein and the nucleic acid molecule into the cell. In a preferred embodiment, the incubation is carried out between 5°C to 40°C, preferably between 15°C and 37°C, more preferably between 17°C and 27°C, and most preferably at room temperature. In another preferred embodiment the cells are incubated at 37°C, preferably at an atmosphere containing 5% CO₂.

Accordingly, described herein is a method for solid phase transfection of at least one protein and at least one nucleic acid molecule into at least one cell, wherein the method comprises the steps of
(a) providing a solid carrier containing at least one spot comprising a transfection reagent and at least one protein and at least one nucleic acid molecule;
(b) bringing the spot in step (a) into contact with at least one cell, and
(b') incubating the at least one cell on the spot.

The above method may further comprise step (a') and/or step (a") as defined above after step (a) and before step (b).

In a preferred embodiment described herein, the at least one cell is incubated for at least 0.5, 1, 2, 3, 4, 6, 8, 10, 12, 18, 24, 36, 48, or 72 hours. In a most preferred embodiment, the incubation is carried out from 3 to 72 hours.

According to a preferred embodiment described herein, the protein and/or the nucleic acid molecule is covalently linked to at least one lipid moiety.

According to another preferred embodiment described herein, the protein and/or the nucleic acid molecule is covalently linked to at least one detectable substance. The detectable substance may be used for detecting the protein and/or the nucleic acid molecule in the cell and/or outside of the cell after transfection. The detection in the cell might be carried out inside the intact cell or after cell lysis. The detection outside of the cell might be carried out at the cell surface or in the liquid being in contact with the cell.

The term "detectable substance" does not exhibit any particular limitation and may be selected from the group consisting of radioactive labels, fluorescent dyes, compounds having an enzymatic activity, magnetic labels, antigens, and compounds having a high binding affinity for a detectable substance. A compound having an enzymatic reactivity such as the enzyme luciferase which produces a light signal upon contact with the respective substrate can also be used as a detectable substance which may be linked covalently to said substrate. Coupling a detectable substance to an antigen allows the detection of the substance by an antibody/enzyme-complex (the enzyme being e.g. phosphatase) catalysing a detectable color reaction when using a suitable substrate. A compound with a high binding affinity for a different detectable substance such as biotin which binds to a detectable substance covalently linked to e.g. streptavidin, is a further possibility for making a substance detectable. In a preferred embodiment, the detectable substance is a substance which can be detected inside of a cell. In a preferred embodiment, the detectable substance is a fluorescent dye.

In one embodiment described herein, the protein and/or the nucleic acid molecule is linked to a detectable substance and the method further comprises a step of detecting the detectable substance after step (b).

Accordingly, described herein is a method for solid phase transfection of at least one protein and at least one nucleic acid molecule, wherein the at least one protein and/or the at least one nucleic acid molecule is linked to a detectable substance into at least one cell, wherein the method comprises the steps of
(a) providing a solid carrier containing at least one spot comprising a transfection reagent and at least one protein and at least one nucleic acid molecule;
(b) bringing the spot in step (a) into contact with at least one cell, and
(c) detecting the detectable substance.

The above method may further comprise step (a') and/or step (a") as defined above after step (a) and before step (b) and/or step (b') as defined above after step (b) and before step (c).

The reaction conditions for detecting the detectable substance according to the present disclosure depend upon the detection method selected. It is within the knowledge of the person skilled in the art to choose the optimal parameters, such as buffer system, temperature and pH for the respective detection system to be used.

The detection of the detectable substance may comprise one or more detection method(s) selected from the group consisting of immunoblotting, immunoprecipitation, immunocapture, monoclonal antibody immobilization of platelet antigens or enzyme linked immuno sorbent assay (ELISA), flow cytometry, protein array technology, spectroscopy, mass spectrometry, chromatography, surface plasmonic resonance, fluorescence extinction and/or fluorescence energy transfer. The detection method for measuring the detectable substance can, for example, be selected from the group consisting of an enzyme assay, a chromogenic assay, a lumino assay, a fluorogenic assay, and a radioimmune assay. The reaction conditions to perform detection of the detectable label depend upon the detection method selected. It is within the knowledge of the person skilled in the art to choose the optimal parameters, such as buffer system, temperature and pH for the respective detection system to be used.

In a preferred embodiment described herein, at least part of the transfection mixture containing the transfection reagent and the at least one protein and the at least one nucleic acid molecule in the at least one spot are dried or frozen. In a preferred embodiment the transfection mixture containing the transfection reagent and the at least one protein and the at least one nucleic acid molecule in the at least one spot are dried or frozen. In a preferred embodiment, the cells to be transfected are seeded straight on the frozen or dried transfection mixture. In another preferred embodiment, the cells to be transfected are seeded on the de-frozen or re-hydrated transfection mixture.

In another preferred embodiment a fraction of the transfection mixture is dried or frozen on at least part of the spots on the solid carrier. In one preferred embodiment the transfection reagent and/or the at least one protein in the at least one spot are dried or frozen. In another preferred embodiment the transfection reagent and/or the at least one nucleic acid molecule in the at least one spot are dried or frozen. In case only a fraction of the transfection mixture is dried or frozen in the at least one spot, the transfection mixture is completed prior to step (a) of the method described herein by providing the remaining fraction of the transfection mixture. For example if the at least one protein is dried or frozen on the at least one spot, then the transfection reagent and the at least one nucleic acid are applied on the at least one spot prior to step (a). In another example, if the at least one protein and the transfection reagent are dried or frozen on the at least one spot, then the at least one nucleic acid is applied on the at least one spot prior to step (a). In another example, if the at least one nucleic acid is dried or frozen on the at least one spot, then the transfection reagent and the at least one protein are applied on the at least one spot prior to step (a). In another example, if the at least one nucleic acid and the transfection reagent are dried or frozen on the at least one spot, then the at least one protein is applied on the at least one spot prior to step (a).

Methods for drying reactants on a solid surface are known in the art. Any suitable method known in the art for drying a reactant to a solid surface, preferably without adding any further component to the reactant, can be used according to the present disclosure. A dried spot is preferably obtained (i) by vacuum centrifugation followed by lyophilisation or (ii) by freeze drying, steps (i) or (ii) are preferably carried out after the spot has been frozen to - 20°C. In a preferred embodiment, a frozen spot is obtained by freezing the solid carrier to at least 0°C, at least -5°C, at least -10°C, at least -15°C, at least -20°C, at least -30°C, or at least -80°C.

Also described herein is a use of a solid carrier, preferably a solid carrier as defined herein, having a spot with a dried or frozen transfection mixture for reverse transfection of at least one protein and at least one nucleic acid.

In a preferred embodiment described herein, the cell is a cell derived from a cell culture or a cell derived from a sample taken from an individual.

The term "individual" as used herein is not limited to any particular individual and may be a fungus, an animal, a human being or a plant.

The sample taken from an individual may be any sample. In one embodiment described herein, the sample may be derived from a healthy or diseased naturally occurring system, preferably a sample containing a body fluid or components derived from a body fluid. In one embodiment, the sample may be a naturally occurring system such as a solution selected from the group consisting of serum, saliva, urine, bile, lymph, tissue, like e.g. bladder or kidney, cerebrospinal fluid and/or other body fluids in the case of an animal or human individual.

In another embodiment the sample may be derived from a healthy or diseased naturally occurring system, preferably a sample containing a tissue or components derived from a tissue. A tissue or a component derived therefrom may be of various origins, like for example leafs, roots, petals, germs or stems in the case of plant individual, or muscle tissue, heart tissue, liver tissue, kidney tissue, epithelial tissue, connective tissue, or nervous tissue.

The term "cell culture" in its various grammatical forms, refers to cells grown in suspension, roller bottles, flasks and the like. Large scale approaches, such as bioreactors, including adherent cells growing attached to microcarriers in stirred fermentors, also are included. Moreover, it is possible to not only to culture surface-dependent cells, but also to use the suspension culture techniques with the inventive media. If the cells are grown on microcarriers, the microcarrier can be selected from the group of microcarriers based on dextran, collagen, plastic, gelatin, membranes in general and cellulose and others.

According to the present disclosure, cells derived from a cell culture may be derived from animals, plants or fungus. Animal cells may be derived for example from insects, fish, birds, or mammals. In a preferred embodiment, cells derived form a cell culture are mammalian cells including those of human origin, which may be primary cells derived from a tissue sample, diploid cell strains, transformed cells or established cell lines. Mammalian cells can include human and non-human cells alike. Mammalian cells of non-human origin can be monkey kidney cells, bovine kidney cells, dog kidney cells, pig kidney cells, rabbit kidney cells, mouse kidney cells, rat kidney cells, sheep kidney cells, hamster kidney cells, Chinese hamster ovarian cells or an animal cell derived from any tissue. In particular, mammalian cells that can cultivated in the culture medium can be BSC-1 cells, LLC-MK cells, CV-1 cells, COS-cells, COS-1 cells, COS-3 cells, COS-7 cells, VERO cells, MDBK cells, MDCK cells, CRFK cells, RAF cells, RK-cells, TCMK-1 cells, LLC-PK cells, PK15 cells, LLC-RK cells, MDOK cells, BHK-21 cells, CHO cells, 293 cells, NS-1 cells MRC-5 cells, WI-38 cells, BHK cells, 293 cells, HeLa, MCF7, MCF10a, RK-cells and stem cells (e.g. iPS).

The sample may be derived from a mammal, preferably a mammal selected from the group consisting of human, mouse, rat, pig, cat, dog, horse, goat, cattle, cow, and monkey and/or others. In a preferred embodiment described herein, the sample is derived from a human. In another embodiment, the sample contains isolated body fluid compounds or processed body fluids derived from a mammal, preferably a mammal selected from the group consisting of human, mouse, rat, pig, cat, dog, horse, goat, cattle, cow, and monkey and/or others. In a preferred embodiment, the sample contains isolated body fluid compounds or processed body fluids derived from a human.

In a preferred embodiment, the sample is pre-purified before step (b) of the method described herein. In a more preferred embodiment, the pre-purification comprises the step of removing impurities that impair significantly or prevent the transfection of the protein into the cell.

According to the present invention, the protein is an antibody or a fragment thereof.

The term "antibody" as used herein may be any antibody including biologically active fragments of an antibody having substantially the same biological function as the antibody, e.g. the ability to bind a specific antigen. In particular, the antibody according to the present invention can be selected from the group consisting of a naturally occurring antibody, a polyclonal antibody, a chimeric antibody, a monoclonal antibody, e.g. derived by conventional hybridoma techniques, and an antibody or antibody fragment obtained by recombinant techniques, e.g. phage display or ribosome display, mutated antibodies and (semi)-synthetic antibodies. For example, the antibody or at least one fragment thereof may contain one or more mutations or variations, such as added, deleted or substituted amino acids or nucleic acids, as long as it has no negative effect on the interaction with the specific antigen. The antibody of the present invention may belong to any immunoglobulin class.

According to the present invention, the term "fragment" means any portion of an antibody as defined above as long as it has the ability to bind to the desired antigen. Moreover, a fragment of the present invention may comprise several different portions from said antigen. Examples of fragments of an antibody antigen binding fragment (FAB), single chain variable fragment (scFv), variable domain of the heavy chain (VH), variable domain of the light chain (VL), complementary determining regions (CDRs), and combinations thereof. The term "scFv" used herein means a fusion of the variable regions of the heavy and light chains of any immunoglobulin, linked together with a linker, such as for example serine, glycine, any other natural or non-natural amino acid, a peptide, a protein, or a nucleic acid.

For example, the antibody or the at least one fragment thereof may be a humanized antibody or at least one fragment thereof derived from the murine antibody H398. There is no limitation as to the technique of humanization of the antibody, as long as the antibody binds to the desired antigen. Examples of humanization include CDR-grafting, The expression "humanized antibody" used herein means any antibody in which protein engineering is used to reduce the amount of foreign ("non-human") protein sequence by swapping e.g. rodent antibody constant regions and/or the variable-domain framework with sequences that are found in human antibodies. In a further embodiment of the antibody fragment as defined above, the at least one fragment is a Fab-region, a scFv, or any post-translationally processed recombinant derivative thereof.

In another preferred embodiment described herein, the protein is a nuclease being involved in genome editing, like for example CAS9, transcription-like effector (TALEN), or zinc-finger nucleases.

In a preferred embodiment described herein, the transfection mixture in the spot comprising a transfection reagent, at least one protein and at least one nucleic acid molecule on the solid carrier, wherein the at least one nucleic acid molecule is selected from the group consisting of at least one small interfering RNA (siRNA) molecule, at least one complementary DNA (cDNA) molecule, at least one short hairpin (shRNA) molecule, at least one viral transfection vector and combinations thereof.

In a more preferred embodiment described herein, molecules of each class, i.e. proteins and nucleic acid molecules, preferably siRNA molecules, cDNA molecules, shRNA molecules, and/or viral transfection vectors, are complexed with a transfection agent before they are mixed into the transfection mixture. In a preferred embodiment, at least one class of molecules is complexed with a transfection agent before mixing the molecules into the transfection mixture. More preferably at least two, three or four classes of molecules are complexed with a transfection agent before mixing the molecules into the transfection mixture. In a most preferred embodiment of the present invention all four classes of molecules are complexed with a transfection agent before mixing the molecules into the transfection mixture. The transfection agent can be the same or different for each class of molecules.

In a most preferred embodiment described herein, each class of molecules is complexed with a different transfection reagent, most preferably with a transfection agent which has been optimized for the transfection of this class of molecules, before the molecules are mixed into the transfection mixture. For example, proteins to be transfected can be complexed with a transfection agent which has been tested to be particularly useful for the transfection of the particular proteins before mixing the proteins into the transfection mixture. Optionally, siRNA molecules, cDNA molecules, shRNA molecules, and/or viral transfection vectors, which are transfected into the cell together with the proteins are complexed with respective transfection agents which have been tested to be particularly useful for the transfection of these particular siRNA molecules, cDNA molecules, shRNA molecules, and/or viral transfection vectors. After that the complexed proteins and optionally the complexed siRNA molecules, cDNA molecules, shRNA molecules, and/or viral transfection vectors are mixed into the transfection mixture.

In a preferred embodiment described herein, the transfection mixture containing the complexed class of molecules does not contain any bovine serum albumin as stabilization agent in protein solutions, e.g. antibody solutions or recombinant protein solution, as it may interfere in the complexation process. Nevertheless pure BSA may be used for the protein transfection, e.g. as a positive control. In another preferred embodiment, the transfection mixture containing the complexed class of molecules contains at least one substance which stabilizes the complexes during vacuum centrifugation or freezing processes or freeze drying processes, preferably a sugar, more preferably trehalose. In another preferred embodiment, the transfection mixture containing the complexed class of molecules contains at least one substance which contribute to an endosomal escape after uptake of the complexed molecule by the cell via endocytosis, preferably the substance is a proton sponge, more preferably a substance selected from the group consisting of tertiary amine groups containing a hydrophobic chain, histidine-rich molecules, and poly (amido amine) polymers.

In a preferred embodiment described herein, the solid carrier has at least two spots and wherein at least two spots comprise a transfection mixture containing at least one different protein and/or at least one different nucleic acid molecule. In a particularly preferred embodiment, the solid carrier has at least two spots and wherein at least two spots comprise a transfection mixture containing at least one different protein and/or at least one different siRNA molecule and/or at least one different cDNA molecule and/or at least one different shRNA molecule and/or at least one different viral transfection vector. Accordingly, described herein is an assay and/or a microarray, wherein the transfection of cells with different protein, siRNA, cDNA shRNA and/or viral vector is carried out in parallel in one experimental setting

In another preferred embodiment described herein, the solid carrier has at least two different spots and wherein at least two spots are brought into contact with at least one different cell type. Accordingly, described herein is an assay and/or a microarray, wherein the transfection of different cells with the same protein and/or the same nucleic acid molecule, preferably siRNA, cDNA shRNA, and/or the same viral vector is carried out in parallel in one experimental setting. Further, described herein is an assay and/or a microarray, wherein the transfection of different cells with a different protein and/or a different nucleic acid molecule, preferably siRNA, cDNA shRNA, and/or a different viral vector is carried out in parallel in one experimental setting.

Described herein is a solid carrier containing at least one spot comprising a transfection reagent and at least one protein and at least one nucleic acid molecule. The solid carrier can be any solid carrier as defined herein, including the definitions given in this application, like for example, the definitions given for solid carrier, protein, antibody, cell, transfection mixture, and/or transfection reagent given herein.

In a preferred embodiment of the solid carrier described herein, the solid carrier contains 9 to 225 spots per cm².

In a preferred embodiment of the solid carrier described herein, each spot has a diameter of 5 to 750 µm.

In a preferred embodiment of the solid carrier described herein, at least one spot on the solid carrier further comprises at least one siRNA molecule and/or at least one cDNA molecule. The siRNA can be any siRNA as defined herein and the cDNA molecule can be any cDNA molecule as defined in this application.

In a preferred embodiment described herein, the spot comprising a transfection reagent, at least one protein and at least one nucleic acid molecule on the solid further contains a glycoprotein, preferably a high-molecular weight glycoprotein, most preferably fibronectin. In a preferred embodiment, the glycoprotein is mixed with gelatin resulting in a mixture as defined herein.

In a preferred embodiment described herein, the spot comprising a transfection reagent, at least one protein and at least one nucleic acid molecule on the solid carrier further contains a sugar, preferably sucrose, and or a cell culture medium as defined herein. In a most preferred embodiment, the spot contains a solution of 0.4 M sucrose in a low serum cell culture medium, most preferably OptiMEM produced by Invitrogen.

In a preferred embodiment described herein, the transfection mixture in the spot comprising a transfection reagent, at least one protein and at least one nucleic acid molecule on the solid carrier are dried or frozen. A dried or frozen transfection mixture is preferably obtained using the methods for drying or freezing described in the present application.

In a preferred embodiment, described herein is a solid carrier as defined herein containing at least one spot comprising a transfection reagent and at least one protein and at least one nucleic acid molecule, wherein the protein is pre-complexed with the transfection reagent. Thus, described herein is a solid carrier, wherein the transfection mixture in the spot comprising a transfection reagent, at least one protein and at least one nucleic acid molecule on the solid carrier contains a protein which is pre-complexed with the transfection reagent

In a preferred embodiment, described herein is a solid carrier containing at least part of the transfection mixture comprising the protein(s) and nucleic acid molecule(s) to be transfected with all necessary transfection reagents and optionally the reagents defined herein in a pre-defined dried or frozen spot, so that it can be stored for weeks, months or years. In a preferred embodiment, the solid carrier described herein comprising a dried or freezed spot is stored for at least two weeks, at least four weeks, at least eight weeks, at least 12 weeks, at least two months, at least three months, at least four months, at least six months, at least one year, at least two years, at least three years before it is brought into contact with a cell and/or a transfection reaction is carried out.

After storage the transfection is initiated by optionally completing the transfection mixture and by bringing cells into contact with the dried or frozen transfection mixture. The process where the transfection mixture is provided first and then the cells are added is called reverse-transfection. Thus, at least part of the transfection mixture can be prepared in advance in a high volume and then brought onto the solid carrier using automated workflows and then be stored, packaged or mailed. This provides a mechanism of automating transfection thereby providing a cost-effective tool which makes a standard transfection mixture available to medical doctors for point-of-care testing or treatment or clinical research or treatment with standardized laboratory protocols.

The solid carrier according to a preferred embodiment described herein, containing spots with one or more dried or frozen standardized transfection mixture(s) can be distributed for world-wide and can be used for a long time, thus minimizing the experiment-to-experiment variations by pipetting deviations which occur any time a transfection mixture is made freshly. Using the invention underlying the present application, it is possible to test or treat the same individual over a period of time repetitively using exactly the same transfection mixture. Further, it is possible to test or treat different individuals simultaneously at different locations all over the world using exactly the same transfection mixture. Accordingly, described herein is a surprising means for the automatization of transfection processes.

Further described herein is a use of the solid carrier as defined herein in the transfection of at least one protein and at least one nucleic acid molecule into at least one cell, preferably using the method for solid phase transfection of at least one protein and at least one nucleic acid molecule into at least one cell described herein.

Described herein is a kit containing solid carrier described herein. The kit may contain e.g. a buffer solution, a solid support as defined above, reaction containers, and/or transfection reagents, when appropriate.

Reverse transfection of proteins and nucleic acids is an exciting enrichment of the technology which has been established by the present invention. A number of available transfection reagents were tested and ranked according their efficiency and influence on cell proliferation. Antibodies, targeting proteins localizing to diverse subcellular structures, were transfected by this approach and shown to label the expected subcellular organelles (e.g., microtubules). For some of them, for instance, antibodies targeting the Golgi marker GM130, a very good co-localization to the endogenous protein over time was demonstrated. Upon the need, cells can be seeded on the prepared substrates and take in the pre-complexed proteins. Accordingly, the present invention provides a cost- and work-efficient technology.

One advantage of the present invention is the fact that in addition to enhanced transfection efficiency, novel classes of assays can be addressed e.g. delay of expression and protein binding.

According to the present invention two transfection set-ups are possible: Nucleic acids and proteins in combination with the same transfection reagent being incubated simultaneously. This results in a one-set-up preparation, enabling cheaper production, less labor and less consumables. On the other hand a set-up is possible, wherein nucleic acids and proteins in combination with different transfection reagents are incubated separately

In contrast to the majority of antibody-based diagnostic and therapy techniques, the present invention is not limited to the antibody-antigen interaction on the cell surface, but enables to work intracellular on the sub-cellular level. Having this tool in hand, cell cultures, primary and, importantly, patient cells can be probed for a number of applications, such as functional screening, molecular diagnostics, testing of drug response, just to mention a few.

The figures show:
Figure 1: Results of Example 1 for the transfection of anti-GM130 AlexaFluor 555 Novagen ProteoJuice + anti-GM130 AlexaFluor 555 (10 µg/ml) after 24 h incubation Scale bar: 50 µm
Figure 2: Results of Example 1 for the transfection of anti-β-Tubulin Cy3 Novagen ProteoJuice + anti-β-Tubulin Cy3 (27.5 µg/ml) after 8 h incubation Scale bar: 50 µm

The present invention will now be further illustrated in the following examples without being limited thereto.

### Examples

### Example 1:

### Transfection Reagent:

- OZ Biosciences DreamFect Gold
- Novagen ProteoJuice

### Antibody:

- anti-GM130 AlexaFluor 555 (5-10 µg/ml)
- anti-â-Tubulin Cy3 (20-50 µg/ml)

### Pipette transfection mix

| | **Volume** |
|---|---|
| OptiMEM/Suc (0.4M) | 2.75 µl |
| Transfection reagent | 1.00 µl |
| Antibody | 2.00 µl |
| → 15- 30 min incubation at RT | |
| 1% Fibronectin in Gelatin (0.2%) | 3.625 µl |
| Total volume | 9.375 µl |

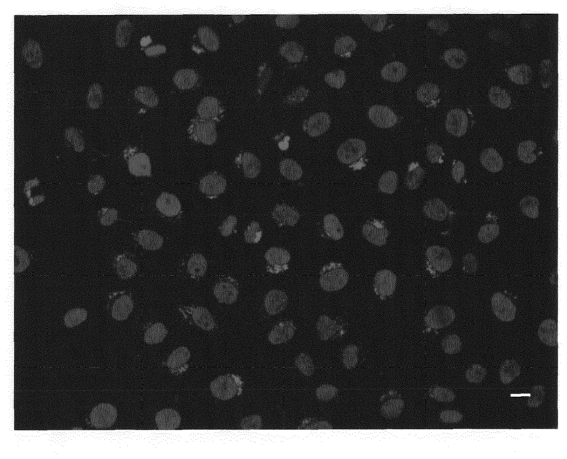

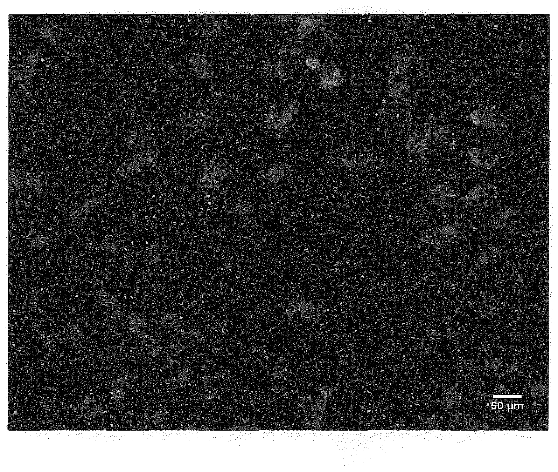

After pipetting OptiMEM/Sucrose (0.4 M), transfection reagent and the antibody, the transfection mix is incubated at room temperature for 15-30 min. Then the Fibronectin/Gelatin mix is added. This transfection mix is then diluted with water in the ratio → transfection mix: water 1 : 10. This solution is then pipetted into multiwell plates (384 well: 5 µl; 96 well: 25 µl; 8 well; 60-100 µl). Now, the plates are either vacuum centrifuged, or frozen down to -20°C with a following lyophilisation step. The result after both methods is a dried transfection solution containing the antibody-transfection reagent complexes ready for transfection. The results of the transfection are shown in Figures 1 and 2.

### Example 2:

### Transfection Reagent for protein complexation:

- Genlantis GeneSilencer
- Novagen ProteoJuice
- PeqFect siRNA

### Transfection Reagent for siRNA complexation:

- Invitrogen Lipofectamine 2000
- Peqlab PeqFect siRNA

### Transfection Reagent for cDNA complexation:

- Peqlab PeqFect siRNA

### Antibody/Protein:

- anti-GM130 AlexaFluor 555 (50 µg/ml)
- goat anti-rabbit IgG Cy3 (100 µg/ml)
- BSA-FITC (1 mg/ml)

### Pipette transfection mix

| | **protein** | **siRNA** | **cDNA** |
|---|---|---|---|
| OptiMEM/Suc (0.4M) | 3.35 µl | 1.50 µl | 2.50 µl |
| Transfection reagent | 0.40 µl | 1.75 µl | 2.25 µl |
| protein | 2.00 µl | | |
| siRNA (30 µM) | | 2.50 µl | |
| cDNA (500 ng/µl) | | | 1.00 µl |

| **→ 15- 30 min incubation at RT** | | | |
|---|---|---|---|
| 1% Fibronectin in Gelatine (0,2%) | 3.625 µl | 3.625 µl | 3.625 µl |
| Total volume | 9.375 µl | 9.375 µl | 9.375 µl |

### Dilution in water (single transfection):

9.375 µL transfection mix + 93.75 µL _{dd}H₂O ? 103.125 µL

### Dilution in water (co-transfection):

9.375 µL transfection mix (e.g. protein) + 9.375 µL transfection mix (e.g. siRNA) + 187.5 µL _{dd}H₂O ? 206.25 µL

After pipetting OptiMEM/Sucrose (0.4 M), transfection reagent and the antibody/siRNA/cDNA, the transfection mix is incubated at room temperature for 15-30 min. Then the Fibronectin/Gelatine mix is added. This transfection mix is then diluted with water in the ratio → transfection mix : water 1 : 10. When a co-transfection is performed, the transfection mixes are one after another added to the double amount of water (187.5 µl) for the dilution. This solution is then pipetted into multiwell plates (384 well: 5 µl; 96 well: 25 µl; 8 well; 60-100 µl). Now, the plates are either spotted on cell arrays, vacuum centrifuged, or frozen down to -20°C with a following lyophilisation step. The result after both methods is a dried transfection solution containing the antibody-transfection reagent complexes ready for transfection.

### Results:

### 1) Transfection efficiency with GeneSilencer and KIF11 siRNA: 31.67%

Transfection efficiency with GeneSilencer and KIF11 siRNA and KIF11 antibody: 47.40%

### 2) Transfection efficiency with Lipofectamine 2000 and KIF11 siRNA: 52.36%

Transfection efficiency with Lipofectamine 2000 and KIF11 siRNA and KIF11 antibody: 68.64%

### 3) Experiment with KIF11 siRNA and goat anti-rabbit Cy3 secondary ab:

Transfection efficiency with Lipofectamine 2000 and KIF11 siRNA: 52.36% Transfection efficiency with Lipofectamine 2000 and KIF11 siRNA and goat anti-rabbit Cy3 secondary antibody: 66.85%

### 4) Other experiment with KIF11 siRNA and BSA-FITC:

Transfection efficiency with Lipofectamine 2000 and KIF11 siRNA: 50.10% Transfection efficiency with Lipofectamine 2000 and KIF11 siRNA and BSA-FITC: 65.01 %

## Claims

1. A method for simultaneous solid phase transfection of at least one protein and at least one nucleic acid molecule into at least one cell, wherein the method comprises the steps of
(a) providing a solid carrier containing at least one spot comprising a transfection reagent, at least one protein and at least one nucleic acid molecule;
(b) bringing the spot in step (a) into contact with at least one cell,
wherein the protein is an antibody or a fragment thereof, and
wherein said solid carrier is produced by
(i-α) coating/spotting at least part of the solid carrier with the at least one nucleic acid molecule and a fraction of the transfection mixture first and then
(ii-α) coating/spotting on the part of the solid carrier being coated/spotted with the at least one nucleic acid molecule and this fraction of the transfection mixture the at least one protein and the remaining fraction of the transfection mixture,
or
(i-β) coating/spotting at least part of the solid carrier with the at least one protein and a fraction of the transfection mixture first and then
(ii-β) coating/spotting on the part of the solid carrier being coated/spotted with the at least one protein and this fraction of the transfection mixture the at least one nucleic acid molecule and the remaining fraction of the transfection mixture.

2. The method of claim 1, wherein the transfection reagent and the at least one protein and/or the at least one nucleic acid molecule in the at least one spot are dried or frozen.

3. The method of claim 1 or 2, wherein the cell is a cell derived from a cell culture or a cell derived from a sample taken from an individual.

4. The method according to any of the above claims, further comprising step (c) incubating the at least one cell on the spot.

5. The method according to claim 4, wherein the at least one cell is incubated for 3 to 72 hours.

6. The method according to any of the above claims, wherein the at least one nucleic acid molecule is selected from the group consisting of siRNA, miRNA, gRNA, cDNA, LNA, and a viral transfection vector, wherein the siRNA molecule optionally contains an antisense strand.

7. The method according to any of the above claims, wherein the solid carrier has at least two spots and wherein at least two spots comprise at least two different proteins and/or at least two different nucleic acid molecules.

8. The method according to any of the above claims, wherein the solid carrier has at least two different spots and wherein at least two spots are brought into contact with at least two different cell types.

9. A solid carrier containing at least one spot comprising a transfection reagent, at least one protein and at least one nucleic acid molecule,
wherein the protein is an antibody or a fragment thereof, and
wherein said solid carrier is produced by
(i-α) coating/spotting at least part of the solid carrier with the at least one nucleic acid molecule and a fraction of the transfection mixture first and then
(ii-α) coating/spotting on the part of the solid carrier being coated/spotted with the at least one nucleic acid molecule and this fraction of the transfection mixture the at least one protein and the remaining fraction of the transfection mixture,
or
(i-β) coating/spotting at least part of the solid carrier with the at least one protein and a fraction of the transfection mixture first and then
(ii-β) coating/spotting on the part of the solid carrier being coated/spotted with the at least one protein and this fraction of the transfection mixture the at least one nucleic acid molecule and the remaining fraction of the transfection mixture.

10. The solid carrier of claim 9, containing 9 to 225 spots per cm².

11. The solid carrier of claim 9 or 10, wherein each spot has a diameter of 5 to 750 µm.

12. The solid carrier according to any of the above claims, wherein the at least one nucleic acid molecule is selected from the group consisting of siRNA, miRNA, gRNA, cDNA, LNA, and a viral transfection vector, wherein the siRNA molecule optionally contains an antisense strand.

13. The solid carrier according to any of claims 9 to 12, wherein the transfection reagent and the at least one protein and/or the at least one nucleic acid molecule in the at least one spot are dried or frozen.

14. Use of the solid carrier according to any of claims 9 to 13 in the transfection of at least one protein into at least one cell.

## Patentansprüche

1. Verfahren zur gleichzeitigen Festphasentransfektion von mindestens einem Protein und mindestens einem Nukleinsäuremolekül in mindestens eine Zelle, wobei das Verfahren die Schritte umfaßt
(a) Bereitstellen eines festen Trägers, enthaltend mindestens eine Stelle, umfassend ein Transfektionsreagens, mindestens ein Protein und mindestens ein Nukleinsäuremolekül,
(b) Inkontaktbringen der Stelle in Schritt (a) mit mindestens einer Zelle,
wobei das Protein ein Antikörper oder ein Fragment davon ist und
wobei der feste Träger hergestellt wird durch
(i-α) zunächst Auftragen/Spotten mindestens eines Teils des festen Trägers mit dem mindestens einen Nukleinsäuremolekül und einem Teil der Transfektionsmischung und dann
(ii-α) Auftragen/Spotten des mindestens einen Proteins und des verbleibenden Teils der Transfektionsmischung auf dem Teil des festen Trägers, auf den das mindestens eine Nukleinsäuremolekül und jener Teil der Transfektionsmischung aufgetragen/gespottet wurde,
oder
(i-β) zunächst Auftragen/Spotten mindestens eines Teils des festen Trägers mit dem mindestens einen Protein und einem Teil der Transfektionsmischung und dann
(ii-β) Auftragen/Spotten der mindestens einen Nukleinsäure und des verbleibenden Teils der Transfektionsmischung auf dem Teil des festen Trägers, auf den das mindestens eine Protein und jener Teil der Transfektionsmischung aufgetragen/gespottet wurde.

2. Verfahren nach Anspruch 1, wobei das Transfektionsreagens und das mindestens eine Protein und/oder das mindestens eine Nukleinsäuremolekül an der mindestens einen Stelle getrocknet oder gefroren werden.

3. Verfahren nach Anspruch 1 oder 2, wobei die Zelle eine Zelle ist, die von einer Zellkultur stammt oder eine Zelle, die von einer Probe stammt, welche von einem Individuum entnommen wurde.

4. Verfahren nach einem der vorstehenden Ansprüche, weiter umfassend Schritt (c) Inkubieren der mindestens einen Zelle an der Stelle.

5. Verfahren nach Anspruch 4, wobei die mindestens eine Zelle für 3 bis 72 Stunden inkubiert wird.

6. Verfahren nach einem der vorstehenden Ansprüche, wobei das mindestens eine Nukleinsäuremolekül ausgewählt ist aus der Gruppe, bestehend aus siRNA, miRNA, gRNA, cDNA, LNA und einem viralen Transfektionsvektor, wobei das siRNA-Molekül gegebenenfalls einen Antisense-Strang enthält.

7. Verfahren nach einem der vorstehenden Ansprüche, wobei der feste Träger mindestens zwei Stellen aufweist und wobei mindestens zwei Stellen mindestens zwei verschiedene Proteine und/oder mindestens zwei verschiedene Nukleinsäuremoleküle umfassen.

8. Verfahren nach einem der vorstehenden Ansprüche, wobei der feste Träger mindestens zwei verschiedene Stellen aufweist und wobei mindestens zwei Stellen in Kontakt mit mindestens zwei verschiedenen Zelltypen gebracht werden.

9. Fester Träger, enthaltend mindestens eine Stelle, umfassend ein Transfektionsreagens, mindestens ein Protein und mindestens ein Nukleinsäuremolekül,
wobei das Protein ein Antikörper oder ein Fragment davon ist und
wobei der feste Träger hergestellt wird durch
(i-α) zunächst Auftragen/Spotten mindestens eines Teils des festen Trägers mit dem mindestens einen Nukleinsäuremolekül und einem Teil der Transfektionsmischung und dann
(ii-α) Auftragen/Spotten des mindestens einen Proteins und des verbleibenden Teils der Transfektionsmischung auf dem Teil des festen Trägers, auf den das mindestens eine Nukleinsäuremolekül und dieser Teil der Transfektionsmischung aufgetragen/gespottet wurde,
oder
(i-β) zunächst Auftragen/Spotten mindestens eines Teils des festen Trägers mit dem mindestens einen Protein und einem Teil der Transfektionsmischung und dann
(ii-β) Auftragen/Spotten der mindestens einen Nukleinsäure und des verbleibenden Teils der Transfektionsmischung auf dem Teil des festen Trägers, auf den das mindestens eine Protein und dieser Teil der Transfektionsmischung aufgetragen/gespottet wurde.

10. Fester Träger nach Anspruch 9, enthaltend 9 bis 225 Stellen pro cm².

11. Fester Träger nach Anspruch 9 oder 10, wobei jede Stelle ein Durchmesser von 5 bis 750 µm aufweist.

12. Fester Träger nach einem der vorstehenden Ansprüche, wobei das mindestens eine Nukleinsäuremolekül ausgewählt ist aus der Gruppe, bestehend aus siRNA, miRNA, gRNA, cDNA, LNA und einem viralen Transfektionsvektor, wobei das siRNA-Molekül gegebenenfalls einen Antisense-Strang enthält.

13. Fester Träger nach einem der Ansprüche 9 bis 12, wobei das Transfektionsreagens und das mindestens eine Protein und/oder das mindestens eine Nukleinsäuremolekül an der mindestens einen Stelle getrocknet oder gefroren sind.

14. Verwendung des festen Trägers nach einem der Ansprüche 9 bis 13 in der Transfektion von mindestens einem Protein in mindestens eine Zelle.

## Revendications

1. Procédé de transfection simultanée en phase solide d'au moins une protéine et d'au moins une molécule d'acide nucléique dans au moins une cellule, dans lequel le procédé comprend les étapes de
(a) la fourniture d'un support solide contenant au moins un dépôt comprenant un réactif de transfection, au moins une protéine et au moins une molécule d'acide nucléique ;
(b) la mise en contact du dépôt dans l'étape (a) avec au moins une cellule,
dans lequel la protéine est un anticorps ou l'un de ses fragments, et
dans lequel ledit support solide est produit par
(i-α) l'enrobage de/le dépôt sur au moins une partie du support solide avec l'au moins une molécule d'acide nucléique et une fraction du mélange de transfection en premier et ensuite
(ii-α) l'enrobage de/le dépôt sur la partie dudit support solide enrobée/avec le dépôt de l'au moins une molécule d'acide nucléique et de cette fraction du mélange de transfection, avec l'au moins une protéine et la fraction restante du mélange de transfection,
ou
(i-β) l'enrobage de/le dépôt sur au moins une partie du support solide avec l'au moins une protéine et une fraction du mélange de transfection en premier et ensuite
(ii-β) l'enrobage de/le dépôt sur la partie du support solide enrobée/avec le dépôt de l'au moins une protéine et de cette fraction du mélange de transfection, avec l'au moins une molécule d'acide nucléique et la fraction restante du mélange de transfection.

2. Procédé selon la revendication 1, dans lequel le réactif de transfection et l'au moins une protéine et/ou l'au moins une molécule d'acide nucléique dans l'au moins un dépôt sont séchés ou congelés.

3. Procédé selon la revendication 1 ou 2, dans lequel la cellule est une cellule dérivée d'une culture cellulaire ou une cellule dérivée d'un échantillon prélevé chez un individu.

4. Procédé selon l'une quelconque des revendications ci-dessus, comprenant en outre l'étape (c) d'incubation de l'au moins une cellule sur le dépôt.

5. Procédé selon la revendication 4, dans lequel l'au moins une cellule est incubée pendant 3 à 72 heures.

6. Procédé selon l'une quelconque des revendications ci-dessus, dans lequel l'au moins une molécule d'acide nucléique est choisie dans le groupe constitué de pARNi, miARN, ARNg, ADNc, LNA, et d'un vecteur viral de transfection, dans lequel la molécule de pARNi contient éventuellement un brin antisens.

7. Procédé selon l'une quelconque des revendications ci-dessus, dans lequel le support solide comporte au moins deux dépôts et dans lequel au moins deux dépôts comprennent au moins deux protéines différentes et/ou au moins deux molécules d'acide nucléique différentes.

8. Procédé selon l'une quelconque des revendications ci-dessus, dans lequel le support solide comporte au moins deux dépôts différents et dans lequel au moins deux dépôts sont mis en contact avec au moins deux types cellulaires différents.

9. Support solide contenant au moins un dépôt comprenant un réactif de transfection, au moins une protéine et au moins une molécule d'acide nucléique,
dans lequel la protéine est un anticorps ou l'un de ses fragments, et
dans lequel ledit support solide est produit par
(i-α) l'enrobage de/le dépôt sur au moins une partie du support solide avec l'au moins une molécule d'acide nucléique et une fraction du mélange de transfection en premier et ensuite
(ii-α) l'enrobage de/le dépôt sur la partie dudit support solide enrobée/avec le dépôt de l'au moins une molécule d'acide nucléique et de cette fraction du mélange de transfection, avec l'au moins une protéine et la fraction restante du mélange de transfection,
ou
(i-β) l'enrobage de/le dépôt sur au moins une partie du support solide avec l'au moins une protéine et une fraction du mélange de transfection en premier et ensuite
(ii-β) l'enrobage de/le dépôt sur la partie du support solide enrobée/avec le dépôt de l'au moins une protéine et de cette fraction du mélange de transfection, avec l'au moins une molécule d'acide nucléique et la fraction restante du mélange de transfection.

10. Support solide selon la revendication 9, contenant 9 à 225 dépôts par cm².

11. Support solide selon la revendication 9 ou 10, dans lequel chaque dépôt a un diamètre de 5 à 750 *µ*m.

12. Support solide selon l'une quelconque des revendications ci-dessus, dans lequel l'au moins une molécule d'acide nucléique est choisie dans le groupe constitué de pARNi, miARN, ARNg, ADNc, LNA, et d'un vecteur viral de transfection, dans lequel la molécule de pARNi contient éventuellement un brin antisens.

13. Support solide selon l'une quelconque des revendications 9 à 12, dans lequel le réactif de transfection et l'au moins une protéine et/ou l'au moins une molécule d'acide nucléique dans l'au moins un dépôt sont séchés ou congelés.

14. Utilisation du support solide selon l'une quelconque des revendications 9 à 13 dans la transfection d'au moins une protéine dans au moins une cellule.
